# EUROPEAN PATENT APPLICATION

(11) **EP 4 206 221 A1**
(43) Date of publication of application: **05.07.2023**
(21) Application number: 21861551.6
(22) Date of filing: 24.08.2021
(51) Int. Cl.: C07K 14/705, C07K 16/28, C12N 15/13, C12Q 1/70, G01N 33/543, G01N 33/569, C12P 21/08

(54) **VIRAL INFECTION DETERMINATION METHOD**

(30) Priority: 25.08.2020 JP 2020141874
(71) Applicant: KURUME UNIVERSITY, Kurume-shi Fukuoka 830-0011 (JP)
(72) Inventor: HOSHINO, Tomoaki, Kurume-shi, Fukuoka 830-0011 (JP)
(74) Representative: Vossius & Partner Patentanwälte Rechtsanwälte mbB
(86) International application number: PCT/JP2021/030976
(87) International publication number: WO 2022/045126

(57) **Abstract**

The disclosure provides a method of determining whether a subject is infected with a virus, comprising (1) testing whether a protein comprising an amino acid sequence of at least a part of the helicase domain of MDA5 protein and having a molecular weight of about 60 kDa is detected in a sample obtained from the subject; and (2) determining the subject is infected with the virus when the protein was detected. The disclosure also provides a monoclonal antibody capable of binding to the protein.

## Description

### CROSS REFERENCE TO RELATED APPLICATION

This application claims benefit of priority to Japanese Patent Application 2020-141874, the entire content of which is incorporated herein by reference.

### BACKGROUND

### Technical Field:

The disclosure relates to a method of determining whether a subject is infected with a virus, as well as a protein and an antibody which may be used in the method.

### Background Art:

Viral RNAs are sensed by receptors of RIG-I like receptor (RLR) family, which comprises three members, RIG-I (DDX58), LGP2 (DHX58), and MDA5 (IFIH1). All three RLRs are expressed in the cytoplasm. MDA5 detects infection by picornaviruses, such as polio and encephalomyocarditis viruses (EMCVs), and recognizes long double-stranded RNAs (dsRNAs). Similarly to other RLRs MDA5 activates NF-κB and interferon-regulatory factor (IRF) through its N-terminal domain having caspase recruitment domains (CARDs). MDA5 can also recognize single-stranded RNA (ssRNA) viruses such as mouse hepatitis virus (MHV), calicivirus and flavivirus family, as well as RNAs derived from DNA viruses. A previous study reported on a pediatric patient having a homozygous missense mutation (K365E) in MDA5 (Non-Patent Literature 2). The patient had a history of recurrent respiratory tract infections caused by ssRNA viruses such as human rhinovirus (HRV), influenza virus, respiratory syncytial virus (RSV), and coronaviruses, DNA viruses (Adenovirus), and bacteria such as *Haemophilus influenza*, *Mycoplasma pneumoniae*, and *Staphylococcus aureus.* Additionally, Non-Patent Literature 3 discloses that MDA5 is essential for protection against infection by coronaviruses, which are positive single-stranded RNA viruses. Taken together, MDA5 plays an important role in protection against infection by dsRNA viruses, ssRNA viruses, DNA viruses, and bacteria. However, the mechanism of the protection involving MDA5 has not been elucidated.

### Non-Patent Literature

Non-Patent Literature 1: Yueh-Ming Loo, et al., Journal of Virology, 82, 335-345, 2008
Non-Patent Literature 2: Ian T. Lamborn et al., The Journal of Experimental Medicine, 214, 1949-1972, 2017
Non-Patent Literature 3: Zachary B. Zalinger et al., Journal of Virology, 89, 12330-12340, 2015

### SUMMARY

An object of the disclosure is to provide a method of determining whether a subject is infected with a virus.

The inventors have found that a protein comprising an amino acid sequence of at least a part of the helicase domain of MDA5 protein and having a molecular weight of about 60 kDa is detected in sera of healthy humans, and the protein is rapidly released from cytoplasm of peripheral blood mononuclear cells when the cells are stimulated with a dsRNA that mimics a virus. Accordingly, the protein may be used as a marker of viral infection.

An aspect of the disclosure provides a method of determining whether a subject is infected with a virus, comprising
(1) testing whether a protein comprising an amino acid sequence of at least a part of the helicase domain of MDA5 protein and having a molecular weight of about 60 kDa is detected in a sample obtained from the subject; and
(2) determining the subject is infected with the virus when the protein was detected.

An aspect of the disclosure provides a monoclonal antibody, comprising
(i) a heavy chain variable region comprising heavy chain CDR1 comprising the amino acid sequence of SEQ ID NO: 4, heavy chain CDR2 comprising the amino acid sequence of SEQ ID NO: 5, and heavy chain CDR3 comprising the amino acid sequence of SEQ ID NO: 6, and
   a light chain variable region comprising light chain CDR1 comprising the amino acid sequence of SEQ ID NO: 7, light chain CDR2 comprising the amino acid sequence of SEQ ID NO: 8, and light chain CDR3 comprising the amino acid sequence of SEQ ID NO: 9, or
(ii) a heavy chain variable region comprising heavy chain CDR1 comprising the amino acid sequence of SEQ ID NO: 12, heavy chain CDR2 comprising the amino acid sequence of SEQ ID NO: 13, and heavy chain CDR3 comprising the amino acid sequence of SEQ ID NO: 14, and
   a light chain variable region comprising light chain CDR1 comprising the amino acid sequence of SEQ ID NO: 15, light chain CDR2 comprising the amino acid sequence of SEQ ID NO: 16, and light chain CDR3 comprising the amino acid sequence of SEQ ID NO: 17,
   or a monoclonal antibody that competes for binding to MDA5 with said monoclonal antibody.

An aspect of the disclosure provides a kit for determining whether a subject is infected with a virus, comprising an antibody capable of binding to a protein comprising an amino acid sequence of at least a part of the helicase domain of MDA5 protein and having a molecular weight of about 60 kDa.

An aspect of the disclosure provides a protein comprising an amino acid sequence of at least a part of the helicase domain of MDA5 protein and having a molecular weight of about 60 kDa.

An aspect of the disclosure provides use of said protein as a marker for determining viral infection.

The disclosure provides a method of determining whether a subject is infected with a virus, as well as a protein and an antibody which may be used in the method.

### BRIEF DESCRIPTION OF DRAWINGS

Fig. 1 shows the amino acid sequences of the heavy chain variable region and light chain variable region of mouse anti-human MDA5 monoclonal antibody (H27). The CDRs are shown in the boxes.
Fig. 2 shows the amino acid sequences of the heavy chain variable region and light chain variable region of mouse anti-human MDA5 monoclonal antibody (H46). The CDRs are shown in the boxes.
Fig. 3 shows mRNA levels of MDA5 in normal human tissues (lung, spleen, pancreas, muscle, and placenta) determined by real-time quantitative-PCR (RT-qPCR).
Fig. 4 shows results of immunohistochemical analysis of normal tonsil and pancreas using mouse anti-human MDA5 monoclonal antibody (H27). bar: 200 µm.
Fig. 5 shows results of Western blotting of normal lung tissues and lung cancer tissues using mouse anti-human MDA5 monoclonal antibody (H27). Lanes 1 and 2: normal lung tissues (50 mg/lane). Lanes 3 and 4: lung cancer tissues (50 mg/lane).
Fig. 6 shows serum levels of soluble MDA5 protein. Serum levels of soluble MDA5 protein in 32 healthy donors were analyzed by high-sensitive sandwich immunoassay systems. The detection limit of the assay was 80 pg/mL.
Fig. 7 shows characteristics of soluble human MDA5 protein. Peripheral blood mononuclear cells (PBMCs) were isolated from peripheral blood. The cells were stimulated with 250 µg/mL polyinosinic-polycytidylic acid sodium salt (poly I:C) at 37°C. Levels of soluble MDA5 protein in the supernatants were determined. Solid line: poly I: C stimulation. Dashed line: control.
Fig. 8 shows results of Western blotting of MDA5 protein. Lane 1: whole PBMCs. Lane 2: Supernatant (0 min). Lane 3: Supernatant, no stimulation (15 min). Lane 4: Supernatant, poly I:C stimulation (15 min). Lane 5: Supernatant, no stimulation (1 hour). Lane 6: Supernatant, poly I:C stimulation (1 hour). Lane 7: Supernatant, no stimulation (2 hours). Lane 8: Supernatant, poly I:C stimulation (2 hours).

### DETAILED DESCRIPTION

Unless otherwise defined, the terms used herein are read as generally understood by those skilled in the technical fields such as organic chemistry, medical sciences, pharmaceutical sciences, molecular biology, and microbiology. Several terms used herein are defined as below. The definitions herein take precedence over the general understanding.

When a numerical value is accompanied with the term "about", the value is intended to represent any value in the range of -10% of the value to +10% of the value. For example, "about 20" means "a value from 18 to 22." A range defined with values of the lower and upper limits covers all values from the lower limit to the upper limit, including the values of the both limits. When a range is accompanied with the term "about", the both limits are read as accompanied with the term. For example, "about 20 to 30" is read as "18 to 33".

MDA5 protein is a member of the RIG-I-like receptor (RLR) family and is a cytoplasmically expressed protein having a molecular weight of about 120 kDa. The MDA5 protein is known to be involved in protection against viral infection by sensing double-stranded RNA (dsRNA) viruses or single-stranded RNA (ssRNA) viruses, or RNAs derived from DNA viruses in cells, and activating NF-κB and interferon regulatory factor (IRF).

In the disclosure, MDA5 protein may be of any species, typically a mammal, e.g., human, mouse, rat, hamster, rabbit, cat, dog, cow, sheep, or monkey, preferably human. Amino acid sequences of MDA5 protein from various species are readily available through known databases. A representative amino acid sequence of human MDA5 is registered with GenBank accession number AF095844.1 (SEQ ID NO: 1). In the disclosure, MDA5 protein includes products of naturally occurring alleles.

In the disclosure, MDA5 protein may comprise an amino acid sequence in which one or several amino acids are deleted, substituted, or added in an original amino acid sequence (e.g., the amino acid sequence of SEQ ID NO: 1), as long as the functions of MDA5 protein, i.e., RNA recognition and NF-xB/IRF activation, are maintained. The "several amino acids" means preferably 2 to 7, more preferably 2 to 5, and most preferably 2 to 3 amino acids. The amino acid substitution is preferably a conservative substitution between similar amino acid residues.

MDA5 protein may comprise an amino acid sequence having at least about 70%, preferably at least about 80%, more preferably at least about 90%, particularly preferably at least about 95%, most preferably at least about 97%, at least about 98%, or at least about 99% sequence identity with an original amino acid sequence (e.g., the amino acid sequence of SEQ ID NO: 1), as long as the functions of MDA5 protein are maintained. Sequence identity may be calculated by a program such as BLAST, e.g., by BLAST set with default parameters.

Sequence identity is determined by comparing two sequences that are optimally aligned with each other throughout the sequences. In the optimal alignment addition or deletion of amino acid(s), e.g., gap, may be incorporated to the sequences to be compared. Sequence identity may be determined by a program provided by public databases (e.g., DDBJ (http://www.ddbj.nig.ac.jp)), such as FASTA, BLAST, or CLUSTAL W. Alternatively, sequence identity may be determined by a commercially available software for sequence analysis, such as Vector NTI (registered trademark) software or GENETYX (registered trademark) ver. 12.

As demonstrated in the examples below, when an animal is infected with a virus, a protein that comprises an amino acid sequence which is a part of MDA5 protein and has a molecular weight of about 60 kDa is secreted into the blood. The molecular weight herein is that measured by SDS-PAGE under a reducing condition. The protein comprises at least a part of the helicase region of MDA5 protein. The helicase region corresponds to positions 306 to 873 of SEQ ID NO: 1. In the disclosure said protein is referred to as "secretory MDA5 protein".

Accordingly, for example, a method comprising the following steps can determine whether a subject is infected with a virus;
(1) testing whether secretory MDA5 protein is detected in a sample obtained from the subject; and
(2) determining the subject is infected with the virus when secretory MDA5 protein was detected.

Alternatively, the method can assist in determining whether a subject is infected with a virus.

The subject may be any species, typically a mammal, e.g., human, mouse, rat, hamster, rabbit, cat, dog, cow, pig, sheep, or monkey, or a bird, e.g., chicken, quail, turkey, duck, or goose, preferably human.

The sample may be a blood, plasma, or serum sample obtained from the subject. A blood sample may be obtained in a usual manner, e.g., from a vein or artery. A plasma or serum sample may be prepared by processing blood in a manner known to those skilled in the art. The process is not limited and may be any clinically acceptable process. Examples include adding an anticoagulant and centrifugation. The sample may be another body fluid obtained from the subject, e.g., saliva, nasal discharge, sputum, pleural fluid, or ascites. Prior to use the obtained sample may be stored at a low temperature during or after the preparation, for example, it may be stored frozen. The obtained sample may also be concentrated or diluted as needed.

Secretory MDA5 protein can be detected by an immunological method using an antibody that binds to secretory MDA5 protein. Examples of immunological methods include enzyme-linked immunosorbent assay (ELISA, e.g., direct, indirect, sandwich, or competitive ELISA), immunochromatography, Western blotting, flow cytometric analysis, and radioisotope immunoassay (RIA). Preferably the method is sandwich ELISA.

In the disclosure, an antibody mean an affinity ligand based on an immunoglobulin backbone. Examples of antibodies include monoclonal and polyclonal antibodies of any origin, including murine, rat, rabbit, goat, human and other antibodies, and chimeric antibodies comprising sequences derived from multiple species, such as partially humanized antibodies, e.g., partially humanized mouse antibodies. A variable region of an antibody usually consists of three complementarity-determining regions (also referred to as CDRs) flanked by four framework regions. In the disclosure, amino acid positions assigned to CDRs and framework regions in a variable region are defined according to Kabat (see Sequences of Proteins of Immunological Interest, National Institute of Health, Bethesda, Md., (1987) and (1991)).

The antibody can be produced by a conventional known method by using secretory MDA5 protein or an antigenic partial peptide of the protein as an immunogen. For example, the polyclonal antibody can be produced by immunizing an animal with the antigen, and the monoclonal antibody can be produced by hybridoma technology. Alternatively, the antibody can be obtained by generating antibodies by using full-length MDA5 protein as an immunogen and selecting an antibody that binds to secretory MDA5 protein. Alternatively, the antibody may be commercially available.

Secretory MDA5 protein can be obtained by a known genetic engineering method, for example, by generating an expression vector comprising a polynucleotide encoding secretory MDA5 protein and expressing it in a cell. Specifically, an expression vector is constructed so that a polynucleotide encoding secretory MDA5 protein is expressed under the control of an expression control region such as an enhancer or promoter, and a host cell is transformed with the expression vector to express secretory MDA5 protein. Thus, the disclosure also provides a polynucleotide encoding secretory MDA5 protein, an expression vector comprising the polynucleotide, and a transformed cell comprising the polynucleotides or the expression vector.

Alternatively, cells expressing full-length MDA5 protein, e.g., peripheral blood mononuclear cells, or cultured cells transformed to express full-length MDA5 protein may be stimulated with polyinosinic-polycytidylic acid sodium salt (poly I:C) to secrete secretory MDA5 protein into the culture supernatant. Secretory MDA5 protein may be collected from the culture supernatant, for example, by using an existing anti-MDA5 antibody.

The antibody may be a fragment or derivative thereof, as long as it can selectively interact with secretory MDA5 protein. Fragments and derivatives of an antibody include, for example, a Fab fragment, which consists of a first constant heavy chain domain (CH1), a constant light chain domain (CL), a variable heavy chain domain (VH), and a variable light chain domain (VL) of a complete immunoglobulin protein; a Fv fragment, which consists of two variable antibody domains, VH and VL; a single chain Fv fragment (scFv), which consists of two VH and VL domains linked by a flexible peptide linker; and a minibody, which is based on a variable heavy chain domain.

The antibody may be labeled. Those skilled in the art can select a suitable label. Non-limiting examples of labels include fluorescent dyes (e.g., fluorescein, rhodamine, phycoerythrin, fluorescamine), chromophore dyes (e.g., rhodopsin), chemiluminescent compounds (e.g., luminal, imidazole), and bioluminescent proteins (e.g., luciferin, luciferase), haptens (e.g., biotin), enzymes (e.g., peroxidase, alkaline phosphatase, beta-lactamase), radioisotopes (e.g., ³H, ¹⁴C, ³²P, ³⁵S, or ¹²⁵I), particles (e.g., metal particles such as a gold particle), and fluorescent semiconductor nanocrystals (quantum dots). Various labels can be attached to desired antibodies by utilizing various chemical reactions known to those skilled in the art, e.g., amine or thiol reactions. Reactive groups other than amine and thiol, e.g., aldehyde, carboxylic acid, and glutamine, may also be used. Alternatively, the antibody may not be labeled. In this case, a labeled secondary antibody that recognizes the anti-secretory MDA5 protein antibody can be further used.

The antibody may be bound to a suitable support. Any support may be used without limitation, as long as it can immobilize an antibody. The support may have any shape and be made of any material. For example, membranes such as nylon membranes, beads, glass, plastic, and metal supports may be mentioned.

In an embodiment, a monoclonal antibody comprising a heavy chain variable region comprising an amino acid sequence having at least 80%, preferably at least 85%, more preferably at least 90%, even more preferably at least 95%, identity with the amino acid sequence of SEQ ID NO: 2, and/or, a light chain variable region comprising an amino acid sequence having at least 80%, preferably at least 85%, more preferably at least 90%, even more preferably at least 95%, identity with the amino acid sequence of SEQ ID NO: 3 may be used. Preferably, the CDRs of the heavy chain variable region and/or the light chain variable region are maintained.

In an embodiment, a monoclonal antibody comprising a heavy chain variable region comprising an amino acid sequence having at least 80%, preferably at least 85%, more preferably at least 90%, even more preferably at least 95%, identity with the amino acid sequence of SEQ ID NO: 10, and/or, a light chain variable region comprising an amino acid sequence having at least 80%, preferably at least 85%, more preferably at least 90%, even more preferably at least 95%, identity with the amino acid sequence of SEQ ID NO: 11 may be used. Preferably, the CDRs of the heavy chain variable region and/or the light chain variable region are maintained.

In an embodiment, a monoclonal antibody comprising a heavy chain variable region comprising the amino acid sequence of SEQ ID NO: 2, and/or, a light chain variable region comprising the amino acid sequence of SEQ ID NO: 3 may be used.

In an embodiment, a monoclonal antibody comprising a heavy chain variable region comprising the amino acid sequence of SEQ ID NO: 10, and/or, a light chain variable region comprising the amino acid sequence of SEQ ID NO: 11 may be used.

In an embodiment, a monoclonal antibody comprising a heavy chain variable region consisting of the amino acid sequence of SEQ ID NO: 2, and/or, a light chain variable region consisting of the amino acid sequence of SEQ ID NO: 3 may be used.

In an embodiment, a monoclonal antibody comprising a heavy chain variable region consisting of the amino acid sequence of SEQ ID NO: 10, and/or, a light chain variable region consisting of the amino acid sequence of SEQ ID NO: 11 may be used.

In an embodiment, a monoclonal antibody comprising a heavy chain variable region comprising the amino acid sequences of CDR1, CDR2, and CDR3 in the amino acid sequence of SEQ ID NO: 2; and/or, a light chain variable region comprising the amino acid sequences of CDR1, CDR2, and CDR3 in the amino acid sequence of SEQ ID NO: 3 may be used.

In an embodiment, a monoclonal antibody comprising a heavy chain variable region comprising the amino acid sequences of CDR1, CDR2, and CDR3 in the amino acid sequence of SEQ ID NO: 10; and/or, a light chain variable region comprising the amino acid sequences of CDR1, CDR2, and CDR3 in the amino acid sequence of SEQ ID NO: 11 may be used.

In an embodiment, a monoclonal antibody comprising a heavy chain variable region comprising CDR1 comprising the amino acid sequence of SEQ ID NO: 4, CDR2 comprising the amino acid sequence of SEQ ID NO: 5, and CDR3 comprising the amino acid sequence of SEQ ID NO: 6, and/or, a light chain variable region comprising CDR1 comprising the amino acid sequence of SEQ ID NO: 7, CDR2 comprising the amino acid sequence of SEQ ID NO: 8, and CDR3 comprising the amino acid sequence of SEQ ID NO: 9 may be used.

In an embodiment, a monoclonal antibody comprising a heavy chain variable region comprising CDR1 comprising the amino acid sequence of SEQ ID NO: 12, CDR2 comprising the amino acid sequence of SEQ ID NO: 13, and CDR3 comprising the amino acid sequence of SEQ ID NO: 14, and/or, a light chain variable region comprising CDR1 comprising the amino acid sequence of SEQ ID NO: 15, CDR2 comprising the amino acid sequence of SEQ ID NO: 16, and CDR3 comprising the amino acid sequence of SEQ ID NO: 17 may be used.

In an embodiment, a monoclonal antibody comprising a heavy chain variable region comprising CDR1 consisting of the amino acid sequence of SEQ ID NO: 4, CDR2 consisting of the amino acid sequence of SEQ ID NO: 5, and CDR3 consisting of the amino acid sequence of SEQ ID NO: 6, and/or, a light chain variable region comprising CDR1 consisting of the amino acid sequence of SEQ ID NO: 7, CDR2 consisting of the amino acid sequence of SEQ ID NO: 8, and CDR3 consisting of the amino acid sequence of SEQ ID NO: 9 may be used.

In an embodiment, a monoclonal antibody comprising a heavy chain variable region comprising CDR1 consisting of the amino acid sequence of SEQ ID NO: 12, CDR2 consisting of the amino acid sequence of SEQ ID NO: 13, and CDR3 consisting of the amino acid sequence of SEQ ID NO: 14, and/or, a light chain variable region comprising CDR1 consisting of the amino acid sequence of SEQ ID NO: 15, CDR2 consisting of the amino acid sequence of SEQ ID NO: 16, and CDR3 consisting of the amino acid sequence of SEQ ID NO: 17 may be used.

In an embodiment, a monoclonal antibody that competes for binding to MDA5 with any of the above-disclosed monoclonal antibodies may be used. Said antibody can be identified by a competitive assay known to those skilled in the art, e.g., a cross-blocking assay, preferably by a competitive ELISA assay. For example, said antibody causes, for example, at least 20%, 30%, 40%, or 50% inhibition in binding of any of the above-disclosed monoclonal antibodies to MDA5 in a competitive assay.

Alternatively, a monoclonal antibody that competes for binding to MDA5 with any of the above-disclosed monoclonal antibodies may be produced by a conventional known method by using a peptide comprising an epitope of any of the above-disclosed monoclonal antibodies as an immunogen.

As demonstrated in the examples below, a monoclonal antibody comprising a heavy chain variable region consisting of the amino acid sequence of SEQ ID NO: 2 and a light chain variable region consisting of the amino acid sequence of SEQ ID NO: 3, and a monoclonal antibody comprising a heavy chain variable region consisting of the amino acid sequence of SEQ ID NO: 10 and a light chain variable region consisting of the amino acid sequence of SEQ ID NO: 11 recognize secretory MDA5 protein, which comprises a part of MDA5 protein of SEQ ID NO: 1. This means secretory MDA5 protein comprises regions corresponding to epitopes of these monoclonal antibodies.

A monoclonal antibody comprising a heavy chain variable region consisting of the amino acid sequence of SEQ ID NO: 10 and a light chain variable region consisting of the amino acid sequence of SEQ ID NO: 11 recognizes the amino acid sequence QILENSLLNL (SEQ ID NO: 18) at positions 415 to 424 of SEQ ID NO: 1 as an epitope. This means secretory MDA5 protein comprises a region corresponding to positions 415 to 424 of SEQ ID NO: 1. Because positions 415 to 424 of SEQ ID NO: 1 is included in the helicase region (positions 306 to 873 of SEQ ID NO: 1) of MDA5 protein, which is responsible for the helicase activity of MDA5 protein, secretory MDA5 protein comprises at least a part of the helicase region of MDA5 protein, i.e., a region corresponding to positions 306 to 873 of SEQ ID NO: 1. The term "region corresponding to positions 415 to 424 of SEQ ID NO: 1" means the region in MAD5 protein that matches the region of positions 415 to 424 of SEQ ID NO: 1 when the amino acid sequence of MAD5 protein and the amino acid sequence of SEQ ID NO: 1 are optimally aligned. The term "optimally aligned" means two sequences are aligned so that the number of matched amino acids is maximized. The term "region corresponding to positions 306 to 873" is similarly defined.

In step (2), when secretory MDA5 protein was detected in step (1), the subject is determined to be infected with a virus.

Examples of viruses include positive single-stranded RNA viruses such as picornaviruses, coronaviruses (e.g., SARS-related coronaviruses (e.g., SARS-CoV, SARS-CoV-2), MERS coronaviruses, murine hepatitis viruses), encephalomyocarditis viruses, rhinoviruses, Japanese encephalitis viruses, hepatitis C viruses, West Nile viruses, and dengue viruses; negative single-stranded RNA viruses such as influenza viruses, Sendai viruses, and vesicular stomatitis viruses; double-stranded RNA viruses such as reoviruses; and DNA viruses such as poxviruses and adenoviruses.

Another aspect of the disclosure provides a kit for determining whether a subject is infected with a virus comprising an antibody capable of binding to secretory MDA5 protein. The antibody may be provided as dissolved in water or a suitable buffer solution, e.g., phosphate buffered saline (PBS), or as lyophilized, and may be provided in a suitable container. Examples of suitable containers include bottles, vials, syringes, test tubes, plates, and membranes. Containers may be made of a variety of materials such as glass and plastic. The kit may further comprise other components and reagents necessary for the detection of secretory MDA5 protein. For example, the kit may further comprise a labeled secondary antibody, a chromogenic substrate, a blocking solution, a wash buffer, an ELISA plate, or a blotting membrane. The kit may further comprise any other material desirable from a commercial and user's perspective, such as a package insert indicating instructions for use.

An aspect of the disclosure provides a method of determining whether a subject is infected with a virus, comprising
(1) obtaining a sample from the subject,
(2) testing whether secretory MAD5 protein is detected in the sample; and
(3) determining the subject is infected with the virus when secretory MAD5 protein was detected.

An aspect of the disclosure provides an antibody capable of binding to secretory MDA5 protein for use in determining whether a subject is infected with a virus.

An aspect of the disclosure provides use of an antibody capable of binding to secretory MDA5 protein for manufacturing a kit for determining whether a subject is infected with a virus.

For example, the disclosure provides the following embodiments.
1. A method of determining whether a subject is infected with a virus, comprising
   (1) testing whether a protein comprising an amino acid sequence of a part of MDA5 protein and having a molecular weight of about 60 kDa is detected in a sample obtained from the subject; and
   (2) determining the subject is infected with the virus when the protein was detected.
2. A method of determining whether a subject is infected with a virus, comprising
   (1) testing whether a protein comprising an amino acid sequence of at least a part of the helicase domain of MDA5 protein and having a molecular weight of about 60 kDa is detected in a sample obtained from the subject; and
   (2) determining the subject is infected with the virus when the protein was detected.
3. The method according to item 1 or 2, wherein the protein comprises a region corresponding to positions 415 to 424 of SEQ ID NO: 1.
4. The method according to any one of items 1 to 3, wherein the virus is an RNA virus.
5. The method according to any one of items 1 to 4, wherein in step (1) the protein is detected by a sandwich ELISA.
6. The method according to any one of items 1 to 5, wherein in step (1) the protein is detected by using a monoclonal antibody comprising (i) or (ii);
   (i) a heavy chain variable region comprising heavy chain CDR1 comprising the amino acid sequence of SEQ ID NO: 4, heavy chain CDR2 comprising the amino acid sequence of SEQ ID NO: 5, and heavy chain CDR3 comprising the amino acid sequence of SEQ ID NO: 6, and
      a light chain variable region comprising light chain CDR1 comprising the amino acid sequence of SEQ ID NO: 7, light chain CDR2 comprising the amino acid sequence of SEQ ID NO: 8, and light chain CDR3 comprising the amino acid sequence of SEQ ID NO: 9,
   (ii) a heavy chain variable region comprising heavy chain CDR1 comprising the amino acid sequence of SEQ ID NO: 12, heavy chain CDR2 comprising the amino acid sequence of SEQ ID NO: 13, and heavy chain CDR3 comprising the amino acid sequence of SEQ ID NO: 14, and
      a light chain variable region comprising light chain CDR1 comprising the amino acid sequence of SEQ ID NO: 15, light chain CDR2 comprising the amino acid sequence of SEQ ID NO: 16, and light chain CDR3 comprising the amino acid sequence of SEQ ID NO: 17,
      or a monoclonal antibody that competes for binding to MDA5 with said monoclonal antibody.
7. The method according to item 6, wherein in step (1) the protein is detected by using the monoclonal antibody comprising (i) or (ii).
8. The method according to item 7, wherein the monoclonal antibody comprises (i) and comprises a heavy chain variable region comprising an amino acid sequence having at least 90% identity with the amino acid sequence of SEQ ID NO: 2 and a light chain variable region comprising an amino acid sequence having at least 90% identity with the amino acid sequence of SEQ ID NO: 3, or
   the monoclonal antibody comprises (ii) and comprises a heavy chain variable region comprising an amino acid sequence having at least 90% identity with the amino acid sequence of SEQ ID NO: 10 and a light chain variable region comprising an amino acid sequence having at least 90% identity with the amino acid sequence of SEQ ID NO: 11.
9. A monoclonal antibody, comprising
   (i) a heavy chain variable region comprising heavy chain CDR1 comprising the amino acid sequence of SEQ ID NO: 4, heavy chain CDR2 comprising the amino acid sequence of SEQ ID NO: 5, and heavy chain CDR3 comprising the amino acid sequence of SEQ ID NO: 6, and
      a light chain variable region comprising light chain CDR1 comprising the amino acid sequence of SEQ ID NO: 7, light chain CDR2 comprising the amino acid sequence of SEQ ID NO: 8, and light chain CDR3 comprising the amino acid sequence of SEQ ID NO: 9, or
   (ii) a heavy chain variable region comprising heavy chain CDR1 comprising the amino acid sequence of SEQ ID NO: 12, heavy chain CDR2 comprising the amino acid sequence of SEQ ID NO: 13, and heavy chain CDR3 comprising the amino acid sequence of SEQ ID NO: 14, and
      a light chain variable region comprising light chain CDR1 comprising the amino acid sequence of SEQ ID NO: 15, light chain CDR2 comprising the amino acid sequence of SEQ ID NO: 16, and light chain CDR3 comprising the amino acid sequence of SEQ ID NO: 17,
      or a monoclonal antibody that competes for binding to MDA5 with said monoclonal antibody.
10. The monoclonal antibody according to item 9, which is a monoclonal antibody comprising (i) or (ii).
11. The monoclonal antibody according to item 10,
   which is a monoclonal antibody comprising (i) and comprising a heavy chain variable region comprising an amino acid sequence having at least 90% identity with the amino acid sequence of SEQ ID NO: 2 and a light chain variable region comprising an amino acid sequence having at least 90% identity with the amino acid sequence of SEQ ID NO: 3, or
   which is a monoclonal antibody comprising (ii) and comprising a heavy chain variable region comprising an amino acid sequence having at least 90% identity with the amino acid sequence of SEQ ID NO: 10 and a light chain variable region comprising an amino acid sequence having at least 90% identity with the amino acid sequence of SEQ ID NO: 11.
12. A kit for determining whether a subject is infected with a virus, comprising an antibody capable of binding to a protein comprising an amino acid sequence of a part of MDA5 protein and having a molecular weight of about 60 kDa.
13. A kit for determining whether a subject is infected with a virus, comprising an antibody capable of binding to a protein comprising an amino acid sequence of at least a part of the helicase domain of MDA5 protein and having a molecular weight of about 60 kDa.
14. The kit according to item 12 or 13, wherein the protein comprises a region corresponding to positions 415 to 424 of SEQ ID NO: 1.
15. The kit according to any one of items 12 to 14, comprising the monoclonal antibody according to any one of items 9 to 11.
16. A protein comprising an amino acid sequence of a part of MDA5 protein and having a molecular weight of about 60 kDa.
17. A protein comprising an amino acid sequence of at least a part of the helicase domain of MDA5 protein and having a molecular weight of about 60 kDa.
18. The protein according to item 16 or 17, comprising a region corresponding to the epitope of the monoclonal antibody according to any one of items 9 to 11.
19. The protein according to any one of items 16 to 18, comprising a region corresponding to positions 415 to 424 of SEQ ID NO: 1.
20. Use of the protein according to any one of items 16 to 19 as a marker for determining viral infection.

The entire contents of the documents cited herein are incorporated herein by reference. The embodiments described above are non-limiting and may be modified without deviating from the scope of the invention as defined by the appended claims. The following non-limitative examples are provided for illustrative purposes only.

### Examples

### Real-time quantitative-PCR

The complementary DNAs (cDNAs) isolated from normal human tissues (lung, spleen, pancreas, muscle, and placenta) were purchased from BioChain (Newark, CA). Real-time quantitative-PCR (RT-qPCR) was performed twice with SYBR Green Mastermix (Qiagen) using primers for human beta-actin and MDA5 (Qiagen, Tokyo, Japan) on an Mx3000p PCR machine (Stratagene, La Jolla, CA). Mean relative expression was calculated using the threshold cycle method, as previously reported (S. Takenaka, et al., Biochem Biophys Res Commun 445 (2014) 597-601; M. Tominaga, et al., Respir Investig 55 (2017) 293-299).

### Establishment of an anti-human MDA5 monoclonal antibody

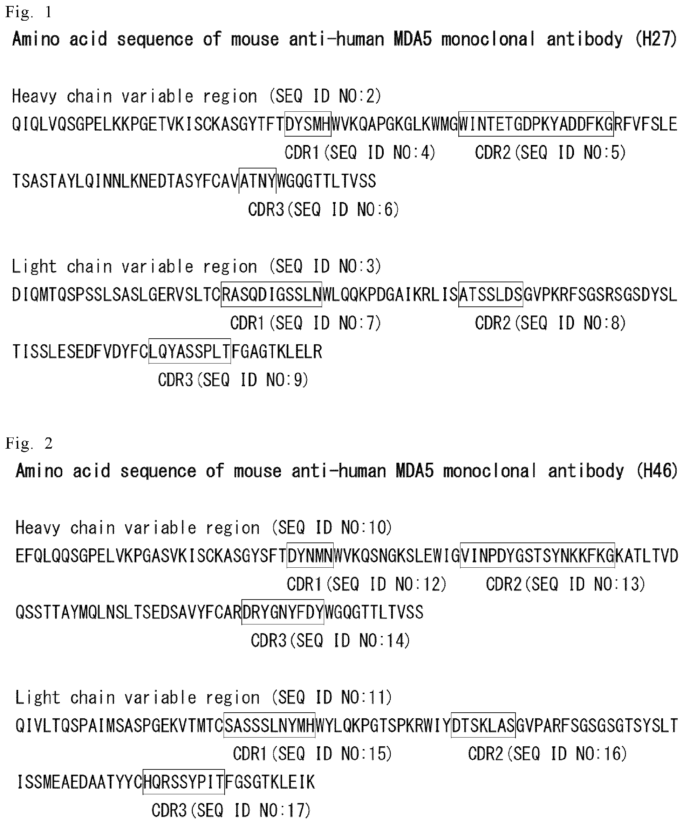

Full-length human MDA5 cDNA (GenBank accession no.: AF095844) with a 6X His tag, GST, and turbo 3C protease cleavage site at the N-terminus was subcloned into the pPSC8 expression vector (Protein Sciences Corporation, Meriden, CT), and designated as pPSC8/human MDA5. Recombinant human MDA5 protein was isolated from SF9 cells cotransfected with baculovirus AcNPV and pPSC8/human MDA5. Anti-human MDA5 monoclonal antibodies (mAbs) were obtained by fusing the mouse myeloma cell line X-63 Ag8/653 with spleen cells isolated from BALB/c mice immunized with the recombinant human MDA5 protein. Anti-human MDA5 mAbs (Clones H27 [mouse IgG1], H46 [mouse IgG2b], H77 [mouse IgG2b], and H85 [mouse IgGG1]) were established. Purified mouse anti-human MDA5 mAbs were generated in our laboratory, as previously reported (Y. Kitasato, et al., Am J Respir Cell Mol Biol 31 (2004) 619-625; S.I. Takenaka, et al., Biochem Biophys Rep 4 (2015) 386-391). The amino acid sequences of the heavy and light chain variable regions of clones H27 and H46 are shown in Figs. 1 and 2, respectively. The epitope of clone H46 analyzed by hydrogen deuterium exchange mass spectrometry (HDX-MS) was the amino acid sequence at positions 415-424 of SEQ ID NO: 1, QILENSLLNL (SEQ ID NO: 18).

### Immunohistochemical staining

Immunohistochemical staining was performed as reported (Y. Kitasato, et al., previously cited; T. Nouno, et al., J Thorac Dis 11 (2019) 4005-4017). Briefly, the lung tissues were fixed with 10% buffered formalin and embedded in paraffin wax. Serial sections (4µm thick) were cut from paraffin-embedded tissues and placed on poly 1-lysine-coated slides. The deparaffinized sections were autoclaved for 3 min in 10 mM citric acid buffer (pH 6.0). Sections were incubated in 0.3% H₂O₂ for 10 min to block endogenous peroxidase activity and then stained with an in-house mouse anti-human MDA5 mAb (H27 [mouse IgG1], 1 µg/mL). Mouse IgG1 (BioLegend, Tokyo, Japan) was used as control. These antibodies were applied to the sections at 4°C for 18 h or at 25°C for 1-2 h. Positive reactivity was identified using goat anti-mouse and anti-rabbit immunoglobulins (Ig) conjugated to a peroxidase-labeled polymer (En Vision Dual Link system-HRP, Agilent Dako, Tokyo, Japan) and a Liquid DAB Substrate/Chromogen System (Agilent Dako).

### Human subjects

Lung tissues and lung cancers were obtained from two patients with squamous carcinoma of the lung who underwent lobectomy at the Kurume University Hospital (a 67-year-old male and a 71-year-old male). Sera and/or urine samples were obtained from 32 healthy donors (23 males and 9 females, aged 28 to 54 years). Formalinfixed paraffin-embedded tissues of the tonsil (4-year-old female) and pancreas (51-year-old female) were purchased from Bio-options, Inc. (Brea, CA). This study was approved by the Institutional Review Board of Kurume University Hospital (approval date: July 31, 2019; approval number: 19090) and was performed in accordance with the 2013 Helsinki Declaration. Informed consent for participation in this study was obtained from all participants.

### PBMC isolation and in vitro stimulation

Peripheral blood mononuclear cells (PBMCs) were isolated from peripheral blood using LymphoprepTM (STEMCELL TECHNOLOGIES, Vancouver, Canada). The cells were washed with cold PBS twice and suspended in cold PBS at a density of 2 × 10⁶ cells/mL. The cells were stimulated with 250 µg/mL polyinosinic-polycytidylic acid sodium salt (poly I:C) (catalog no.: P1530, Merk, Tokyo, Japan) at 37°C. The supernatants were harvested at various time points.

### Western blotting analysis

Western blotting analysis was performed by using NuPAGE (registered trademark) 7% Tris-Acetate Midi Gel (Thermo Fisher Scientific, Tokyo, Japan) according to the manufacture's protocol. Anti-human MDA5 mAb (clone H27 or clone H46, 1 µg/mL) was used for immunoblotting, as previously reported (T. Hoshino, et al., Am J Respir Crit Care Med 176 (2007) 49-62).

### Establishment of human MDA5 sandwich immunoassay system

The human MDA5 sandwich immunoassay system was established using electrochemiluminescence in a MESOSCALE DISCOVERY (registered trademark) assay system (Meso Scale Japan, Tokyo, Japan). Briefly, mouse anti-human MDA5 monoclonal antibody (clone H46) was used as the primary mAb, dissolved at 2 µg/mL in phosphate-buffered saline (PBS), dispensed into plates in aliquots of 25 µL/well, and left undisturbed overnight at 4°C. The plates were then washed three times with 150 µL Quantikine Wash Buffer 1 (R&D Systems, Minneapolis, MN, USA). Block Ace blocking solution (100 µL/well; Nacalai Tesque, Kyoto, Japan) was added and incubated for at least one hour at room temperature to prevent nonspecific adhesion of the secondary antibody to the plates. The plates were then washed thrice. The samples were aliquoted at 25 µL/well. Recombinant human MDA5 protein diluted to 50 ng/mL, 10 ng/mL, 2 ng/mL, 400 pg/mL, 80 pg/mL, 16 pg/mL, and 3.2 pg/mL was used as a standard. After 1 h of incubation at room temperature, each well was washed thrice. Next, 2 µg/mL biotin-labeled mouse anti-human MDA5 secondary mAb (clone H27) was added at 25 µL/well, followed by incubation for 1 h at room temperature after which each well was washed three times. This was followed by the addition of 50 µL of 0.5 µg/mL MSDSULFO-TAG (trademark) labeled streptavidin to each well, and the plates were left for 30 min at room temperature. Each well was washed three times. The amount of human MDA5 protein was measured using MESO QuickPlex SQ 120 according to the manufacturer's protocol.

### Results

### Characteristics of MDA5 expression in tissues

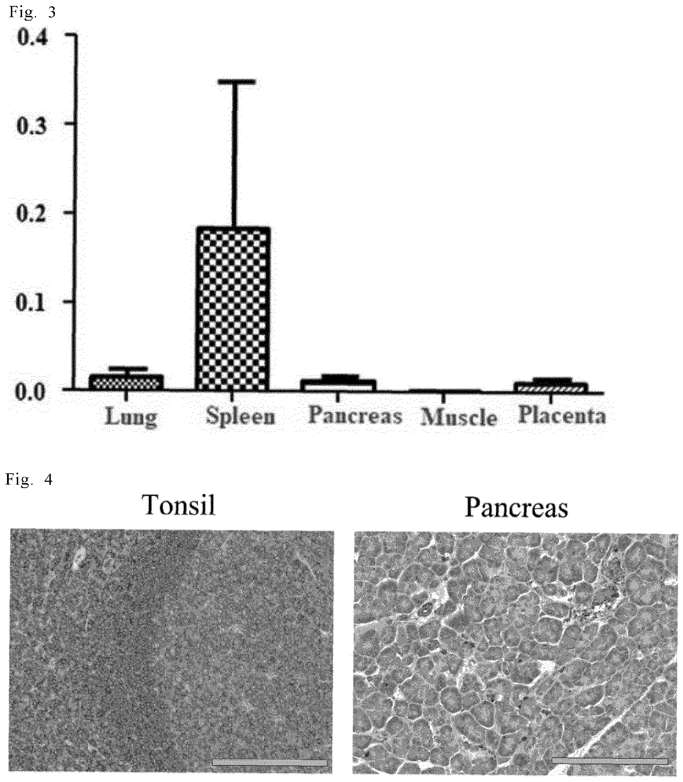

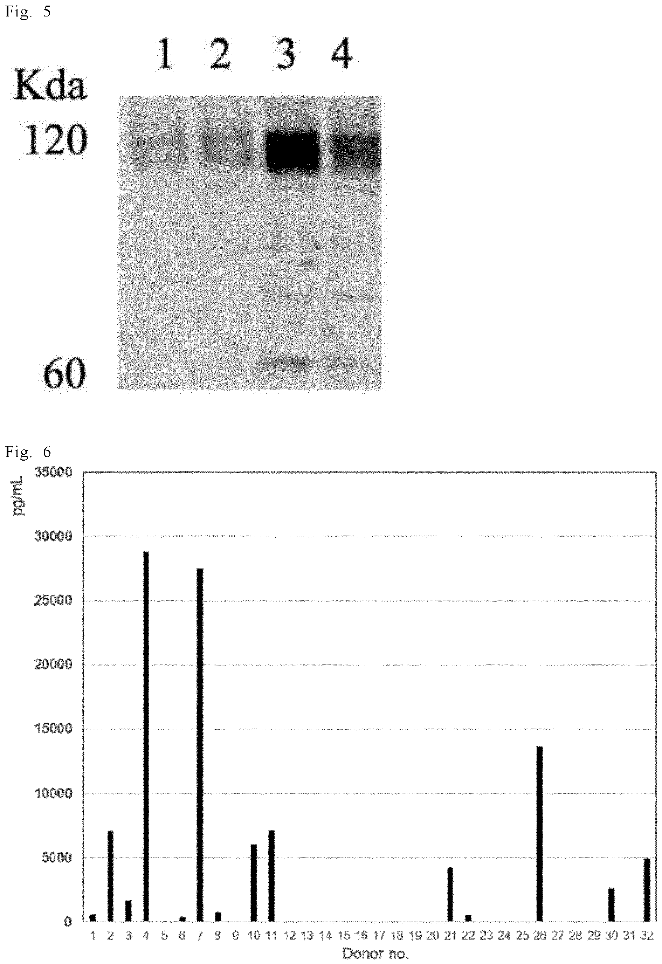

To study mRNA levels of MDA5 in normal human tissues, real-time quantitative-PCR was performed. Repeated analysis revealed that MDA5 mRNA was constitutively expressed in the lung, spleen, pancreas, and placenta and was weakly expressed in the muscle (Fig. 3). We obtained normal tonsil and pancreas. Immunohistochemical analysis was performed to analyze MAD5 protein expression using in-house anti-MDA5 mAb clone H27. MDA5 protein was strongly expressed in the cytoplasm of lymphoid cells in the tonsil and pancreatic cells in the pancreas (Fig. 4). We obtained normal lung tissues and lung cancer tissues from two patients with squamous carcinoma of the lung. Both normal lung tissues and lung cancers constitutively and strongly expressed the MDA5 protein. The molecular size of human MDA5 is about 120 kDa, as previously reported (D.C. Kang, et al., Proc Natl Acad Sci U S A 99 (2002) 637-642). Moreover, we found a weak band of the MDA5 protein of about 60 kDa in both normal and cancerous lung tissues (Fig. 5). The same results were obtained by using two anti-human MDA5 mAbs (clone H27 or clone H46).

### Soluble MDA5 protein in sera

We assumed that there was soluble form of human MDA5 protein in human sera. Initially, we established in-house Enzyme-Linked Immuno Sorbent Assay (ELISA) system using two human MDA5 mAbs (clones H27 and H46), as we previously reported (S.I. Takenaka, et al., previously cited). The detection range was normally 400 pg/mL to 25 ng/mL. We analyzed MDA5 protein in sera of 7 healthy donors by using the in-house MDA5 ELISA. MDA5 protein was detectable (395 pg/mL) in the sera of one of 7 donors. However, 395 pg/mL was near limit of detectable level of this ELISA assay. Therefore, we established in-house high-sensitive sandwich immunoassay systems by using electrochemiluminescence in MESOSCALE DISCOVERY (registered trademark) assay system (Meso Scale Japan, Tokyo, Japan), and analyzed MDA5 protein in the sera of 32 healthy donors (Fig. 6). The detection range of this system was normally 80 pg/mL to 50 ng/mL. Soluble MDA5 protein was detectable in sera from 14 donors (3314 ± 7215 pg/mL, n = 32). The levels of soluble MDA5 protein in the sera of three donors were above 10 ng/mL. In contrast, soluble MDA5 was not detectable in the sera of 18 donors. Interestingly, soluble MDA5 protein was not detectable in the urine of 6 healthy donors. This suggests that normal kidneys do not excrete about 60 kDa soluble MDA5 through nephron.

### Characteristics of soluble MDA5 protein

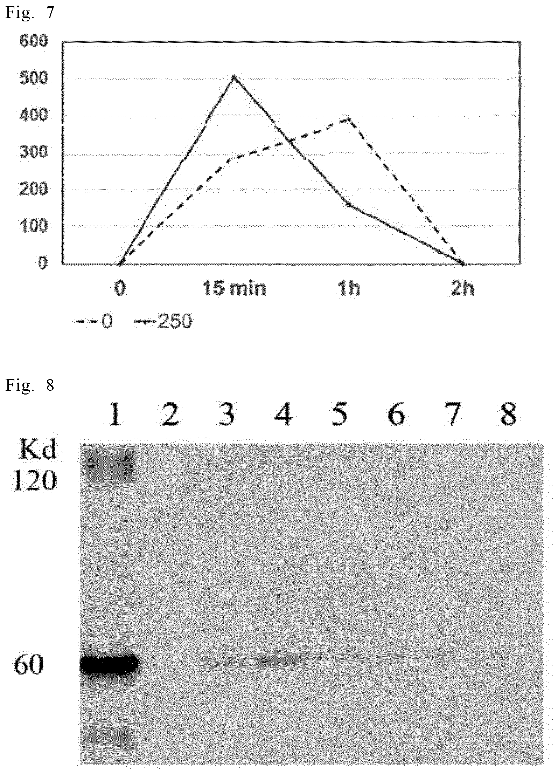

Immunohistochemical analysis of the tonsil showed lymphoid cells strongly express MDA5. We also found soluble form of human MDA5 protein in sera from healthy donors. Accordingly, we hypothesized that PBMCs could produce soluble MDA5 protein. Subsequently, PBMCs were isolated from four healthy donors. Because RPMI1640 with 10% FCS stimulated PMBCs, PBMCs were suspended in cold PBS at 2 × 10⁶ cells/mL. PBMCs were stimulated with 250 µg/mL polyinosinic-polycytidylic acid sodium salt (poly I:C) at 37°C. MDA5 detects poly I:C, a synthetic dsRNA analogue (H. Kato, et al., Nature 441 (2006) 101-105). The supernatants were harvested at various time points. The high-sensitive sandwich immunoassay system revealed that the levels of MDA5 protein increased in the supernatant of PMBCs within 15 min after poly I:C stimulation. The levels of MDA5 protein were decreased in the supernatant of PMBCs after 30 min. They were barely detectable after 2 hours. Representative data are shown in Fig. 7. Western blotting showed the same results; MDA5 protein was increased in the supernatant of PMBCs within 15 min after poly I:C stimulation but was hardly detectable after 2 h. The molecular size of the soluble MDA5 was about 60 kDa. Interestingly, PBMCs expressed MDA5 protein with molecular weights of about 60 and 120 kDa (Fig. 8). In this study, we also found weak bands of MDA5 protein around 60 kDa in both normal and cancerous lung tissues (Fig. 5). Full-length MDA5 (IFIH1) cDNA encodes 1025 amino acids (D.C. Kang, et al., previously cited) with a predicted molecular weight of 116,686.83. These results suggest that soluble MDA5 protein can be generated by alternative splicing of MDA5 mRNA or digestion of MDA5 protein by proteases in some tissues.

### Industrial Applicability

By using the method of the disclosure whether a subject is infected with a virus can be determined, and the obtained results can be used for diagnosis.

## Claims

1. A method of determining whether a subject is infected with a virus, comprising
(1) testing whether a protein comprising an amino acid sequence of at least a part of the helicase domain of MDA5 protein and having a molecular weight of about 60 kDa is detected in a sample obtained from the subject; and
(2) determining the subject is infected with the virus when the protein was detected.

2. The method according to claim 1, wherein the protein comprises a region corresponding to positions 415 to 424 of SEQ ID NO: 1.

3. The method according to claim 1 or 2, wherein the virus is an RNA virus.

4. The method according to any one of claims 1 to 3, wherein in step (1) the protein is detected by a sandwich ELISA.

5. The method according to any one of claims 1 to 4, wherein in step (1) the protein is detected by using a monoclonal antibody comprising (i) or (ii);
(i) a heavy chain variable region comprising heavy chain CDR1 comprising the amino acid sequence of SEQ ID NO: 4, heavy chain CDR2 comprising the amino acid sequence of SEQ ID NO: 5, and heavy chain CDR3 comprising the amino acid sequence of SEQ ID NO: 6, and
a light chain variable region comprising light chain CDR1 comprising the amino acid sequence of SEQ ID NO: 7, light chain CDR2 comprising the amino acid sequence of SEQ ID NO: 8, and light chain CDR3 comprising the amino acid sequence of SEQ ID NO: 9,
(ii) a heavy chain variable region comprising heavy chain CDR1 comprising the amino acid sequence of SEQ ID NO: 12, heavy chain CDR2 comprising the amino acid sequence of SEQ ID NO: 13, and heavy chain CDR3 comprising the amino acid sequence of SEQ ID NO: 14, and
a light chain variable region comprising light chain CDR1 comprising the amino acid sequence of SEQ ID NO: 15, light chain CDR2 comprising the amino acid sequence of SEQ ID NO: 16, and light chain CDR3 comprising the amino acid sequence of SEQ ID NO: 17,
or a monoclonal antibody that competes for binding to MDA5 with said monoclonal antibody.

6. The method according to claim 5, wherein in step (1) the protein is detected by using the monoclonal antibody comprising (i) or (ii).

7. The method according to claim 6, wherein the monoclonal antibody comprises (i) and comprises a heavy chain variable region comprising an amino acid sequence having at least 90% identity with the amino acid sequence of SEQ ID NO: 2 and a light chain variable region comprising an amino acid sequence having at least 90% identity with the amino acid sequence of SEQ ID NO: 3, or
the monoclonal antibody comprises (ii) and comprises a heavy chain variable region comprising an amino acid sequence having at least 90% identity with the amino acid sequence of SEQ ID NO: 10 and a light chain variable region comprising an amino acid sequence having at least 90% identity with the amino acid sequence of SEQ ID NO: 11.

8. A monoclonal antibody, comprising
(i) a heavy chain variable region comprising heavy chain CDR1 comprising the amino acid sequence of SEQ ID NO: 4, heavy chain CDR2 comprising the amino acid sequence of SEQ ID NO: 5, and heavy chain CDR3 comprising the amino acid sequence of SEQ ID NO: 6, and
a light chain variable region comprising light chain CDR1 comprising the amino acid sequence of SEQ ID NO: 7, light chain CDR2 comprising the amino acid sequence of SEQ ID NO: 8, and light chain CDR3 comprising the amino acid sequence of SEQ ID NO: 9, or
(ii) a heavy chain variable region comprising heavy chain CDR1 comprising the amino acid sequence of SEQ ID NO: 12, heavy chain CDR2 comprising the amino acid sequence of SEQ ID NO: 13, and heavy chain CDR3 comprising the amino acid sequence of SEQ ID NO: 14, and
a light chain variable region comprising light chain CDR1 comprising the amino acid sequence of SEQ ID NO: 15, light chain CDR2 comprising the amino acid sequence of SEQ ID NO: 16, and light chain CDR3 comprising the amino acid sequence of SEQ ID NO: 17,
or a monoclonal antibody that competes for binding to MDA5 with said monoclonal antibody.

9. The monoclonal antibody according to claim 8, which is the monoclonal antibody comprising (i) or (ii).

10. The monoclonal antibody according to claim 9, which is a monoclonal antibody comprising (i) and comprising a heavy chain variable region comprising an amino acid sequence having at least 90% identity with the amino acid sequence of SEQ ID NO: 2 and a light chain variable region comprising an amino acid sequence having at least 90% identity with the amino acid sequence of SEQ ID NO: 3, or
which is a monoclonal antibody comprising (ii) and comprising a heavy chain variable region comprising an amino acid sequence having at least 90% identity with the amino acid sequence of SEQ ID NO: 10 and a light chain variable region comprising an amino acid sequence having at least 90% identity with the amino acid sequence of SEQ ID NO: 11.

11. A kit for determining whether a subject is infected with a virus, comprising an antibody capable of binding to a protein comprising an amino acid sequence of at least a part of the helicase domain of MDA5 protein and having a molecular weight of about 60 kDa.

12. A protein comprising an amino acid sequence of at least a part of the helicase domain of MDA5 protein and having a molecular weight of about 60 kDa.

13. Use of the protein according to claim 12 as a marker for determining viral infection.
